# EUROPÄISCHE PATENTSCHRIFT

(11) **EP 1 294 919 B1**
(45) Veröffentlichungstag und Bekanntmachung des Hinweises auf die Patenterteilung: **19.07.2006**
(21) Anmeldenummer: 01931696.7
(22) Anmeldetag: 15.05.2001
(51) Int. Cl.: C12N 15/87, C12N 5/10

(54) **VERFAHREN IN VITRO ZUM EINBRINGEN VON NUKLEINSÄURE IN EINE ZELLE (TRANSFEKTION) MITTELS CALCIUMPHOSPHAT**
IN VITRO METHOD FOR INTRODUCING A NUCLEIC ACID INTO A CELL (TRANSFECTION) BY MEANS OF CALCIUM PHOSPHATE
PROCEDE IN VITRO D'INTRODUCTION D'ACIDE NUCLEIQUE DANS UNE CELLULE (TRANSFECTION) GRACE A DU PHOSPHATE DE CALCIUM

(30) Priorität: 17.05.2000 DE 10024334
(43) Veröffentlichungstag der Anmeldung: 26.03.2003
(73) Patentinhaber: MediGene AG, 82152 Planegg/Martinsried (DE)
(72) Erfinder: HÖRER, Markus, 82152 Planegg (DE); REHBERGER, Bernd, 86368 Gersthofen (DE); MOEBIUS, Ulrich, 82131 Gauting (DE)
(74) Vertreter: Bösl, Raphael Konrad
(86) Internationale Anmeldenummer: PCT/EP2001/005531
(87) Internationale Veröffentlichungsnummer: WO 2001/088171

(56) Entgegenhaltungen:
- WO-A-96/07750
- DATABASE BIOSIS [Online] BIOSCIENCES INFORMATION SERVICE, PHILADELPHIA, PA, US; 1987 CHEN C ET AL: "HIGH-EFFICIENCY TRANSFORMATION OF MAMMALIAN CELLS BY PLASMID DNA" Database accession no. PREV198784087230 XP002177320 in der Anmeldung erwähnt & MOLECULAR AND CELLULAR BIOLOGY, Bd. 7, Nr. 8, 1987, Seiten 2745-2752, ISSN: 0270-7306
- WILSON STEVEN P ET AL: "Optimization of calcium phosphate transfection for bovine chromaffin cells: Relationship to calcium phosphate precipitate formation." ANALYTICAL BIOCHEMISTRY, Bd. 226, Nr. 2, 1995, Seiten 212-220, XP002177319 ISSN: 0003-2697 in der Anmeldung erwähnt
- DATABASE WPI Section Ch, Week 198837 Derwent Publications Ltd., London, GB; Class B04, AN 1988-264283 XP002177321 & US 7 156 579 A (US DEPT HEALTH & HUMAN SERVICE) 2. August 1988 (1988-08-02)

## Beschreibung

Die vorliegende Erfindung betrifft ein in vitro Verfahren zum Einbringen von Nukleinsäure in wenigstens eine Zelle (Transfektion) mittels Calciumphosphat (CaPi), wobei zunächst die Nukleinsäure mit einer Reaktionslösung, die Ca²⁺ und PO₄³⁻ - Ionen aufweist, vermischt wird, anschließend die Reaktionslösung inkubiert wird, wodurch ein Präzipitat gebildet wird, das wenigstens die Nukleinsäure, Calcium und Phosphat aufweist, anschließend das Präzipitat zu der Zelle zugegeben wird und die Zelle nochmals inkubiert wird, wodurch die Nukleinsäure in die Zelle aufgenommen wird. Weiterhin betrifft die vorliegende Erfindung ein Verfahren zur Herstellung der transfizierten Zelle.

Mit zunehmendem Interesse an der Aufklärung der Struktur und Funktion von Genen und Proteinen im Rahmen der Gen- und Biotechnologie wurden weltweit in den Labors verschiedenste Methoden entwickelt, Nukleinsäuren in frisch isolierte oder in kultivierte Zellen einzuführen. In Abhängigkeit von der zu untersuchenden Fragestellung können Zellen mit von außen zugegebener Nukleinsäure (externe Nukleinsäure) behandelt werden und diese dauerhaft entweder in das Genom integrieren oder als ringförmiges extrachromosomales Nukleinsäuremolekül aufweisen. In beiden Fällen wird die externe Nukleinsäure vor der Zellteilung vermehrt (repliziert) und auf die Tochterzelle weitergegeben, weshalb diese Transfektionsmethode auch als stabile Transfektion bezeichnet wird. Alternativ dazu können Zellen externe Nukleinsäure nur vorübergehend aufnehmen (transiente Transfektion), wobei die zugeführte Nukleinsäure nicht vermehrt und daher bei den folgenden Zellteilungen nicht gezielt auf die Tochterzellen weitergegeben wird und je nach Kopienanzahl mehr oder minder schnell verloren geht. Transient transfizierte Zellen werden in der Regel wenige Stunden oder Tage nach der Transfektion aufgeschlossen (geerntet bzw. lysiert). Daran anschließend werden in weiteren Laborversuchen beispielsweise die Funktion der auf der externen Nukleinsäure kodierten Proteine, die nach der Transfektion in der Zelle exprimiert wurden, untersucht.

Unabhängig von dem Verfahren, das zur Einführung von Nukleinsäure in die Zelle verwendet wird, ist die Effizienz sowohl bei der stabilen als auch bei der transienten Transfektion sehr stark von dem verwendeten Zelltyp abhängig. Zellen die sich bereits für einen längeren Zeitraum in Kultur befinden (Zellinien) unterscheiden sich teilweise um ein Vielfaches in ihrer Fähigkeit externe Nukleinsäure aufzunehmen und die darauf kodierten Proteine zu exprimieren. So kann eine Transfektionsmethode, die für einen Zelltyp etabliert wurde und zu guten Transfektionseffizienzen führt bei einem anderen Zelltyp vollkommen ungeeignet sein.

Eines der ältesten Verfahren zum Einführen von Nukleinsäuren in Zellen verwendet das Polysaccharid DEAE―Dextran als Trägersubstanz für die externe Nukleinsäure. DEAE-Dextran wurde ursprünglich als Mediator für die Einführung von Ribonukleinsäure (RNA) des Poliovirus (Vaheri und Pagano (1965) Virology 27:434) sowie von Desoxiribonukleinsäure (DNA) des Simian Virus (SV40) und von Polyomavirus (McCutchan und Pagano (1968) J. Natl. Cancer Inst. 41:351; Warden und Thorne (1968) J. Gen. Virol. 3:371) in Zellen verwendet. Die Methode wird mit geringfügigen Modifikationen auch heute noch für die Transfektion von viralen Genomen und ringförmigen DNA-Molekülen (Plasmiden), die virale Gensequenzen tragen, angewandt. Obwohl der exakte Wirkmechanismus von DEAE-Dextran nicht bekannt ist, wird angenommen, daß das Polymer die externe Nukleinsäure, in den meisten Fällen externe DNA, bindet und so den Angriff von zellulären Nukleinsäure-zerstörenden Enzymen (Nukleasen) verhindert und/oder selbst an die Oberfläche von Zellen bindet und dadurch die Aufnahme von DNA in die Zelle mittels Endozytose begünstigt. DEAE-Dextran vermittelte Transfektionen werden gewöhnlicher Weise ausschließlich für die transiente Expression von klonierten Genen und nicht für die stabile Transfektion von Zellen verwendet. Etablierte Zellinien wie beispielsweise BSC-1, CV-1 und COS könne mit DEAE-Dextran sehr gut transfiziert werden, während andere etablierte Zellinien wie beispielsweise HeLa-Zellen wahrscheinlich aufgrund der Toxizität des Polymers ungeeignet sind. Für die Transfektion mit DEAE-Dextran werden in der Regel geringe Mengen an externer DNA verwendet, wobei maximale Transfektionseffizienzen von 10⁵ Zellen mit nur 100 - 200 ng Plasmid-DNA erzielt wurden. Größere Mengen an DNA (< 2 - 3 µg) zeigen keinen gesteigerten sondern einen inhibitorischen Effekt. Im Gegensatz zu anderen Transfektionsverfahren, die im folgenden noch beschrieben werden, die hohe Konzentrationen von DNA benötigen, wird für die DEAE-Dextran vermittelte Transfektion keine zusätzliche Träger-DNA wie beispielsweise Lachs- oder Heringssperma-DNA zu der externen, zu transfizierenden DNA zugegeben. Obwohl dieses Transfektionsverfahren bereits vor über 30 Jahren entwickelt wurde, wird es in den meisten Fällen nur mit geringfügigen Modifikationen des ursprünglich beschriebenen Verfahrens durchgeführt. Mittlerweile ist jedoch bekannt, daß sowohl die Konzentration des verwendeten DEAE-Dextrans als auch die Zeit, für die die Zellen dem DNA/DEAE-Dextran-Gemisch ausgesetzt sind (Inkubationszeit), kritisch ist für den Erfolg der Transfektion.

Da sich nicht alle etablierten Zellinien mit Hilfe von DEAE-Dextran effizient transfizieren lassen, wurden alternative Transfektionsmethoden entwickelt. In einem weiteren Verfahren wird das Polykation Polybren als Mediator vor allem für die Einführung von DNA in Zellen verwendet, die sich gegenüber DEAE-Dextran als resistent erwiesen haben (Kawai und Nishizawa (1984) Mol. Cell. Biol. 4:1172; Chaney et al. (1986) Somatic Cell Mol. Genet. 12:237). Im Gegensatz zur DEAE-Dextran vermittelten Transfektion eignet sich Polybren nicht nur für die transiente sondern auch für die stabile Transfektion von Zellen. Allerdings ist dieses Transfektionsverfahren auf Nukleinsäuren mit geringem Molekulargewicht, beispielsweise Plasmid-DNA beschränkt.

Daneben kann klonierte DNA durch Fusion von Protoplasten, die aus Bakterien gewonnen werden, welche die interessierende Plasmid-DNA tragen, mit den kultivierten Zellen in diese eingeführt werden (Schaffner (1980) Proc. Natl. Acad. Sci. 77:2163; Rassoulzadegan et al. (1982) Nature 295:257). Für dieses Verfahren werden Bakterien in Anwesenheit von Chloramphenicol kultiviert, um die Plasmid-DNA, die die Bakterien in ihrem Inneren in hoher Kopienzahl tragen, zu amplifizieren. Anschließend werden die Bakterien mit dem Enzym Lysozym behandelt, wodurch ihre Zellwand zerstört wird. Die so erhaltenen Protoplasten werden mit den zu transfizierenden Zellen vermischt, wobei das Polymer Polyethylenglycol (PEG) zugesetzt wird, um die Fusion von Zelle und bakteriellem Protoplast zu begünstigen. Während dieses Vorgangs gelangt sowohl die Plasmid-DNA als auch die DNA des bakteriellen Chromosoms in die zu transfizierende Zelle, wobei auf einem bisher nicht aufgeklärten Weg die Plasmid-DNA in den Zellkern der Zelle befördert wird. Anschließend wird PEG entfernt und die Zellen mit frischem Zellkulturmedium versetzt, welches ein Antibiotikum, in der Regel Kanamycin enthält, um das Wachstum von überlebenden Bakterien zu verhindern. Die Methode der Protoplastenfusion wurde sowohl für die transiente Expression von klonierten Genen als auch für die Etablierung von Säugetierzellinien verwendet. Wenn Bakterien und Säugerzellen in einem Verhältnis von 10.000 : 1 gemischt werden, wurden Säugerzellen mit einer Effizienz von ca. 6 % transient transfiziert (Schaffner (1980) supra). Damit ist das Verfahren der Protoplastenfusion im Vergleich zu anderen Transfektionsmethoden vergleichsweise ineffizient, es eignet sich jedoch besonders für das Einführen von DNA in Zellinien, die gegenüber der DEAE-Dextran-vermittelten Endozytose von DNA resistent sind. Weiterhin wurde beobachtet, daß die Protoplastenfusion sehr häufig zur Integration von mehreren Kopien der Plasmid-DNA in das Wirtschromosom führt (Robert de Saint Vincent et al. (1981) Cell 27:267).

Die Verwendung eines kurzen, elektrischen Stromstoßes mit hoher Spannung wurde ebenfalls verwendet, um DNA in eine Vielzahl von Bakterien, Pflanzen- und Säugetierzellen einzuführen (Neumann et al. (1982) EMBO J. 1:841; Wong und Neumann (1982) Biochem. Biophys. Res. Commun. 107:584; Potter et al. (1984) Proc. Natl. Acad. Sci. 81:7161; Fromm et al. (1985) Proc. Natl. Acad. Sci. 82:5824, Fromm et. al. (1986) Nature 319:791; Andreason und Evans (1988) BioTechniques 6:650). Dieses Transfektionsverfahren wird als Elektroporation bezeichnet (Neumann et al. (1982) supra). Dieses Verfahren ist sowohl für die transiente als auch für die stabile Transfektion von Zellen geeignet, jedoch unterscheiden sich die erzielten Transfektionseffizienzen in den einzelnen Berichten teilweise um ein Vielfaches. Durch den Stromstoß werden kleinste Poren in der Größenordnung von wenigen Nanometern in der Plasmamembran der zu transfizierenden Zelle erzeugt (Neumann et al. (1982) supra; Zimmermann (1982) Biochim. Biophys. Acta 694:227). Die externe DNA wird entweder direkt durch diese Poren oder infolge einer Umorganisation der Membrankomponenten, die den Wiederverschluß der entstandenen Poren begleitet, in das Zytoplasma der Zelle aufgenommen. Im Gegensatz zur DEAE-Dextran vermittelten Transfektion oder zur Protoplastenfusion führt die Elektroporation in der Regel zu Zellinien, die eine und nur gelegentlich mehrere Kopien der externen DNA aufweisen (Boggs et al. (1986) Exp. Hematol. 14:988). Die Transfektionseffizienz wird gerade bei der Elektroporation durch eine Vielzahl von Faktoren beeinflußt. Als besonders kritisch wurden dabei die Stärke des angelegten elektrischen Feldes (Patterson (1979) Methods Enzymol. 58:141), die Länge des elektrischen Impulses (Rabussay et al. (1987) Bethesda Res. Lab. Focus 9(3):1), die Temperatur (Reiss et al. (1986) Biochem. Biophys. Res. Commun. 137:244; Chu et al. (1987) Nucleic Acids Res., 15:1311), die Konformation und Konzentration der externen DNA (Neumann et al. (1982) supra; Potter et al. (1984) supra) sowie die ionische Zusammensetzung des Mediums (Rabussay et al. (1987) supra) erkannt. Obwohl mit Hilfe der Elektroporation u.U. sehr hohe Transfektionseffizienzen erzielt werden können, zeigt die Vielzahl der kritischen Parameter, daß gerade dieses Verfahren sehr exakt auf die experimentellen Bedingungen eingestellt werden muß. Die benötigten Vorversuche zur Optimierung der Transfektion durch Elektroporation werden zusätzlich erschwert durch die Tatsache, daß verschiedenste Elektroporationsgeräte im Handel erhältlich sind, die sich bezüglich der einstellbaren Parameter deutlich unterscheiden.

Ein alternatives Verfahren zur Einführung von externer Nukleinsäure in Zellen beruht auf der direkten Mikroinjektion von DNA in den Zellkern (Capecchi (1980) Cell 22:479). Dieses Verfahren besitzt den Vorteil, daß die externe DNA nicht mit zellulären Kompartimenten in Kontakt kommt, wie beispielsweise Endosomen mit niedrigem pH-Wert, der die eingeführte DNA chemisch modifizieren kann. Da aber im Rahmen der Mikroinjektion jede Zelle einzeln mit DNA transfiziert werden muß, eignet sich dieses Verfahren keinesfalls für eine großtechnische Anwendung, die aber für biochemische Analysen gefordert wird. Die Verwendung der Mikroinjektion als Transfektionsverfahren bleibt daher auf die Anwendung im Forschungslabor beschränkt und wird nur angewendet, wenn spezielle Fragestellungen dies erfordern.

Für die Einführung von externer Nukleinsäure in Zellen wurden weiterhin artifizielle Membranvesikel (Liposomen) intensiv untersucht, wobei festgestellt wurde, daß diese Transportvesikel in vitro und in vivo geeignet sind, interessierende DNA in Zellen einzuführen. Bei diesem Verfahren wird die externe DNA oder RNA aufgrund ihrer negativen Gesamtladung in Liposomen eingeschlossen. Anschließend werden die Liposomen mit den zu transfizierenden Zellen vermischt, wodurch die Außenseite der Liposomen durch ihre Ähnlichkeit mit der zellulären Membran mit dieser verschmilzt und die Nukleinsäure in das Innere der Zielzelle befördert wird (Übersichtsartikel: Mannino und Gould-Fogerite (1988) BioTechniques 6:682). Obwohl dieses Verfahren sowohl einfach durchführbar ist als auch hinsichtlich der Transfektionseffizienz zu guten Ergebnissen führt, kann es aufgrund des hohen Preises der benötigten Liposomen nicht im großtechnischen Maßstab verwendet werden.

Das mit Abstand am häufigsten verwendete Verfahren zur Einführung von externer Nukleinsäure in Zellen ist jedoch die Calciumphosphat (CaPi)-vermittelte Transfektion. Bereits im Jahre 1973 wurde durch Graham und van der Eb festgestellt, daß die Aufnahme von DNA in kultivierte Zellen deutlich gesteigert werden kann, wenn diese als Calciumphosphat-DNA-Co-Präzipitat vorliegt (Graham und van der Eb (1973) Virology 52: 456). Bei diesen initialen Versuchen wurde Adenovirus und SV40 DNA in Zellen eingeführt, die im festen Kontakt mit dem Boden des Kultivierungsgefäßes wuchsen (adhärente Zellen). Hierfür wurde Calcium in einer Konzentration von 125 mM und DNA in einer Konzentration von 5 bis 30 µg/ml verwendet, wobei die optimale Bildung eines CaPi-DNA-Co-Präzipitates bei einem neutralen pH-Wert von 7,05 beobachtet wurde. Der genaue Mechanismus der CaPi-vermittelten DNA Transfektion ist ähnlich wie bei DEAE-Dextran unbekannt, es wird jedoch angenommen, daß die transfizierte DNA mittels Endozytose in das Zytoplasma der Wirtszelle aufgenommen und von dort in den Zellkern befördert wird. Zusätzlich zur Bestimmung der kritischen Konzentrationsbereiche von Calcium und DNA hatten Graham und van der Eb die optimale Reaktionszeit zur Ausbildung des CaPi-DNA-Co-Präzipitates auf 20 - 30 min und die anschließende Inkubation der Zellen mit dem Co-Präzipitat auf 5 - 24 h festgelegt. Diese initialen Arbeiten legten den Grundstein für die Entwicklung eines effizienten Transfektionsverfahrens, welches durch verschiedenste Labors in nunmehr über 30 Jahren vielfältigst modifiziert wurde. Eine zusätzliche Steigerung der Transfektionseffizienz wurde beispielsweise durch die Einführung eines zusätzlichen Glycerolschocks (Parker und Stark (1979) J. Virol. 31:360) und/oder einer Chloroquinbehandlung (Luthman und Magnusson (1983) Nucleic Acids Res. 11:1295) erreicht. Ebenfalls wurde die Verwendung von Natriumbutyrat als vorteilhaft für die Proteinexpression von auf Plasmiden kodierten Genen, die einen SV40 enhancer aufweisen, beschrieben (Gormann et al. (1983a) Nucleic Acids Res. 11:7631; Gormann et al. (1983b) Science 221:551).

Im Rahmen der vielfältigen Modifikationen der CaPi-vermittelten Transfektion von Zellen wurde die Behandlung mit Chloroquin, welches die Degradation der externen DNA durch lysosomale Hydrolasen verhindert mit der Anwendung eines Glycerolschocks, welcher die Aufnahme der externen DNA in die Zellen steigert, kombiniert. Abhängig vom Typ der Ausgangszellen können mit Hilfe von CaPi routinemäßig 90 % der kultivierten Zellen gleichzeitig transfiziert werden. Daher ist die CaPi-vermittelte Transfektion häufig die Methode der Wahl sowohl für die transiente Expression von externer DNA in einer großen Anzahl von Zellen als auch für die Etablierung von stabilen Zellinien, die nach der Transfektion integrierte Kopien der externen DNA aufweisen.

Die Schrift US 7,156,579 (veröffentlicht am 2. August 1988) offenbart die Transformation von Säugerzellen, welche die Calciumphosphat-vermittelte Transfektion unter Kontrolle bestimmter kritischer Parameter einschließlich der Zugaberate von Calciumphosphat, der pH-Wertes und des CO₂-Spiegels während der Inkubation umfasst, wobei mit diesem Verfahren stabil transformierte Zelllinien mit einer Effizienz von 10-50 % hergestellt werden können.

Die CaPi-vermittelte Transfektion ist besonders für die effiziente Transfektion von Säugetierzellen geeignet, welche die externe DNA nach der Transfektion stabil über viele Zellkulturpassagen exprimieren (Chen und Okayama (1987) Mol. Cell. Biol. 7:2745). Im Rahmen dieses Protokolls wird das CaPi-DNA-Co-Präzipitat langsam und stufenweise während der Inkubation mit den zu transfizierenden Zellen im Zellkulturmedium auf den Zellen ausgebildet. Für eine effiziente Transfektion wurde ein pH-Wert des verwendeten Puffers von 6,95 und eine zirkuläre DNA-Konzentration von 20 - 30 µg pro ca. 10⁶ Zellen als kritisch erkannt. Diese Ergebnisse konnten jedoch nur mit ringförmig geschlossener DNA (Plasmid-DNA) erzielt werden, wohingegen lineare DNA mit Hilfe dieses Verfahrens nicht in Zellen transfiziert werden konnte. Die Autoren verwendeten für die CaPi-vermittelte Transfektion exponentiell wachsende Zellen, die vor der Transfektion trypsinisiert wurden und anschließend in einer Zelldichte von ca. 5 x 10⁵ Zellen pro 75 cm² in Zellkulturschalen ausgesät wurden. Die Zellen wurden über Nacht mit 10 ml eines Standardnährkulturmediums inkubiert. Am darauffolgenden Tag wurden 10 - 30 µg der interessierenden Plasmid-DNA mit 0,5 ml 0,25 M CaCl₂, 0,5 ml 2 x BES-gepufferter Salzlösung (2 x BBS), welches 50 mM BES (pH 6,95) 280 mM NaCl und 1,5 mM Na₂HPO₄ enthielt, für 10 - 20 min bei Raumtemperatur inkubiert. Das sich in dieser Zeitspanne ausbildende CaPi-DNA-Co-Präzipitat wurde in einer Menge von 1 ml tropfenweise in das Zellkulturmedium der Zellen gegeben. Die Zellkulturschalen wurden anschließend leicht geschwenkt, um die CaPi-DNA Mischung gleichmäßig im Medium zu verteilen. Es folgte ein Inkubationsschritt für 15 - 24 h bei 35°C in einer 2 - 4 % CO₂ Atmosphäre. Danach wurde das Medium entfernt, und die Zellen wurden zweimal in Nährkulturmedium gewaschen, anschließend mit frischem Nährkulturmedium versetzt und nochmals für weitere 24 h bei 35 - 37°C unter einer 5 % CO₂ Atmosphäre inkubiert. Die Zellen wurden für die Selektion von stabilen Transformanten in einem Verhältnis von 1:10 auf neue Zellkulturgefäße verteilt.

Obwohl die Autoren mit diesem Verfahren erstmals sowohl eine Erniedrigung der Temperatur der ersten Inkubationsphase von herkömmlich 37°C auf 35°C sowie die Erniedrigung der CO₂ Atmosphäre von herkömmlich 5 % auf 2 - 4 %, vorzugsweise 3 % CO₂ (Temperatur Shift; CO₂ Shift) beschrieben haben, bleibt die Anwendung ihres Verfahrens auf den Labormaßstab beschränkt und eignet sich nicht für die großtechnische Anwendung.

In den in der Literatur beschriebenen Verfahren wird generell davon ausgegangen, daß in Kultur befindliche Zellen unter Nährkulturmedium und einer stark angereicherten CO₂ Atmosphäre gehalten werden (Taylor et. al. (1973) In Vitro 7(5):295; Minshull und Strong (1985) Int. J. Biochem. 17(4):529). Luft, die als Gasgemisch die Erde als Hülle umgibt besteht neben ca. 77 % Stickstoff, 21 % Sauerstoff aus ca. 1,2 % Wasserdampf, 0,9 % Argon, 0,01 % Wasserstoff und 0,03 % Kohlendioxid (CO₂). (Der kleine Brockhaus Enzyklopädie, 2-bändig (1950) Wiesbaden, Deutschland).

Eine gegenüber normaler Luft ca. 100 - 300-fach erhöhte CO₂ Konzentration (3 - 10 % CO₂) wird als essentiell für ein effizientes Wachstum von Zellen unter Zellkulturbedingungen erachtet, die in einem Zellkulturinkubator durch externe CO₂ Zuführung bereitgestellt werden (Chen und Okayama, supra; Chen und Okayama (1988) BioTechniques 6:632; Wilson (1995) Analytical Biochemistry 226:212).

In der WO 96/07750 ist ebenfalls ein Verfahren zur effizienten Transfektion von eukaryotischen Wirtszellen mit DNA durch CaPi-vermittelte Transfektion beschrieben. Obwohl in dieser Internationalen Patentanmeldung ein Verfahren beschrieben ist, mit dem möglichst große CaPi-DNA-Co-Präzipitatpartikel erhalten werden, wird auch hier von Standardkulturbedingungen hinsichtlich der verwendeten CO₂ Atmosphäre (5 % CO₂) ausgegangen. Als besonders kritisch werden dagegen für die CaPi-vermittelte Transfektion die Konzentration von Ca²⁺ und PO₄³⁻, der pH-Wert und die Temperatur (35°C) erachtet. Dem gegenüber kann eine Reduktion der CaPi-DNA-Co-Präzipitatpartikel erreicht werden, indem der pH-Wert des Nährkulturmediums langsam aufgrund des durch die Zellen produzierten CO₂ (Bildung von HCO₃⁻ durch das im Nährkulturmedium befindliche CO₂ und H₂O) sowie die endogene Milchsäureproduktion der Zellen erniedrigt wird. Die so erreichte kleine Partikelgröße des CaPi-DNA-Co-Präzipitats eignet sich jedoch nur für die Transfektion von bestimmten Zelltypen, so daß dieses Verfahren für Standard-Zellinien nicht generell anwendbar ist. Obwohl diese Internationale Patentanmeldung die großtechnische Anwendung der CaPi-vermittelten DNA Transfektion in Zellen in einem bis zu 40 1 Bioreaktor beschreibt, muß auch in diesen externes CO₂ eingeführt werden, um ein effizientes Zellwachstum zu erlauben.

Damit gehen alle der zahlreichen im Stand der Technik beschriebenen CaPi-DNA Transfektionsverfahren von einer externen CO₂ Begasung der kultivierten Zellen aus, wofür spezielle Inkubationsräume (Brutschränke, Bioreaktoren) verwendet werden, die einen regulierbaren CO₂ Einlaß besitzen. Gerade für die großtechnische Anwendung müssen aber speziell unter dem Kostenaspekt einfache und wenig aufwendige Reaktionsbedingungen geschaffen werden.

Der vorliegenden Erfindung liegt daher die Aufgabe zugrunde, ein Verfahren zur effizienten Einbringung von Nukleinsäure in eine Zelle durch CaPi-vermittelte Transfektion zu schaffen, das kostengünstig im großtechnischen Maßstab durchgeführt werden kann.

Es wurde nun überraschenderweise gefunden, daß Nukleinsäure in Zellen mittels CaPi-vermittelter Transfektion effizient eingebracht werden kann, wobei die Zellen im geschlossenen Kultursystem ohne externe CO₂ Begasung kultiviert werden.

Ein Gegenstand der vorliegenden Erfindung ist daher ein in vitro Verfahren zum Einbringen von wenigstens einer Nukleinsäure in wenigstens eine Zelle mittels Calciumphosphat (CaPi), das die Schritte umfaßt:
(a) Vermischen der genannten Nukleinsäure oder Nukleinsäuren mit mindestens einer Reaktionslösung, die Ca²⁺ und PO₄³⁻ -Ionen aufweist;
(b) Inkubieren der Reaktionlösung(en), wodurch ein Präzipitat gebildet wird, das wenigstens Nukleinsäure, Calcium und Phosphat aufweist;
(c) Zugeben des Präzipitats zu der genannten Zelle;
(d) Inkubieren der Zelle aus Schritt (c) unter Luft im geschlossenen System ohne zusätzliches Kohlendioxid (CO₂), wodurch die Nukleinsäure oder Nukleinsäuren in die Zelle aufgenommen wird.

In einer weiteren bevorzugten Ausführungsform werden zwei oder mehrere verschiedene Nukleinsäuren, insbesondere zwei oder drei verschiedene Nukleinsäuren nacheinander transfiziert. Dabei ist insbesondere bevorzugt, daß bei einer erneuten Transfektion frisches Medium verwendet wird.

Dadurch daß das Inkubieren in Schritt (d) unter Luft im geschlossenen System ohne zusätzliches Kohlendioxid (CO₂) erfolgt, wird insbesondere erreicht, daß das CaPi-vermittelte Transfektionsverfahren im großtechnischen Maßstab unter einfachen und kostengünstigen Bedingungen durchgeführt werden kann.

Unter den Bezeichnungen "Calciumphosphat (CaPi)-vermittelte Transfektion" und "CaPi-DNA-Co-Präzipitat" werden im Sinne der vorliegenden Erfindung die Einführung von externer Nukleinsäure in eine Wirtszelle mit Hilfe eines Co-Präzipitats, welches aus Ca²⁺ und PO₄³⁻ -Ionen sowie der einzuführenden Nukleinsäure besteht, verstanden. Das Präzipitat weist dabei Hydroxyapatit (mit der nährungsweise chemischen Formel von (Ca₅OH(PO₄)₃)₂) auf.

Als externe Nukleinsäure kann sowohl jede Art von RNA (mRNA, rRNA, etc.) als auch jede Art von DNA, vorzugsweise jedoch genomische DNA, cDNA, lineare DNA oder Plasmid-DNA verwendet werden. Besonders bevorzugt ist die Verwendung von zirkulär geschlossener Plasmid-DNA, die nach Standardverfahren aus transformierten Bakterien gewonnen wird.

Die Nukleinsäure, die vorzugsweise Plasmid-DNA ist, wird im Rahmen der vorliegenden Erfindung in einer Menge von ca. 0,2 µg-20 µg, vorzugsweise von ca. 1 µg - 20 µg, insbesondere von ca. 2 µg - 10 µg, vor allem aber von ca. 6 µg pro ca. 10⁶ Zellen verwendet. Innerhalb dieser eingesetzten Mengen werden hohe Transfektionseffizienzen erzielt.

In einer besonders bevorzugten Ausführungsform können mit Hilfe des erfindungsgemäßen Verfahrens gleichzeitig oder nacheinander zwei oder mehrere verschiedene externe Nukleinsäuren, insbesondere zwei oder drei verschiedene externe Nukleinsäuren transfiziert werden. Hierzu werden die interessierenden Nukleinsäuren gemischt und für die Bildung des CaPi-DNA-Co-Präzipitats verwendet. Besonders bevorzugt ist dabei die Verwendung von zwei oder drei verschiedenen Plasmid-DNAs (Co-Transfektion).

Von besonderem Vorteil ist es weiterhin, den pH-Wert der Reakionslösung in Schritt (b) bei Raumtemperatur zwischen ca. 6,8 - 7,1, vorzugsweise zwischen ca. 6,86 - 7,05, insbesondere auf ca. 7,05 einzustellen, wodurch die größte Transfektionseffizienz erzielt wird.

Es ist ebenfalls bevorzugt, das Inkubieren der Reaktionslösung in Schritt (b) bei Raumtemperatur (ca. 18 - 24°C) für ca. 5 - 30 min, vorzugsweise für ca. 10 - 20 min, insbesondere für ca. 15 min durchzuführen. Hierbei werden Partikelgrößen des CaPi-DNA-Co-Präzipitates erhalten, die sich als besonders vorteilhaft für eine effiziente Transfektion erwiesen haben.

In einer weiteren besonders bevorzugten Ausführungsform erfolgt das Inkubieren der Zellen in Schritt (d) bei ca. 37°C im Wärmeschrank für ca. 20 - 72 h, vorzugsweise ca. 30 - 72 h, insbesondere ca. 24 - 48 h, vor allem ca. 40 - 48 h. Dieser Zeitraum hat sich als optimal erwiesen, damit sich die Zellen von der Behandlung mit CaPi-DNA-Co-Präzipitat und der erfolgten Aufnahme der externen Nukleinsäure "erholen" können, bevor sie für weitere Untersuchungen der externen Nukleinsäure bzw. der Expression von auf der Nukleinsäure kodierten Genen verwendet werden.

In einer weiteren Ausführungsform verbleibt die aufgenommene externe Nukleinsäure dauerhaft in der transfizierten Zelle (stabile Transfektion). Sie wird hierbei beispielsweise als episomales, extrachromosomales DNA Partikel in der Zelle durch einen geeigneten Replikationsursprung vermehrt und bei der Zellteilung kontrolliert auf die Tochterzelle weitergegeben.

Alternativ dazu kann die eingeführte externe Nukleinsäure auch, wenn sie keinen Replikationsursprung aufweist, gerichtet oder ungerichtet in das Genom der Zelle integrieren, wodurch sie ebenfalls stabil erhalten bleibt, vor der Zellteilung zusammen mit dem Genom der Wirtszelle repliziert und anschließend bei der Zellteilung an die Tochterzelle weitergegeben wird.

In einer alternativen Ausführungsform bleibt die aufgenommene externe Nukleinsäure nur in der transfizierten Zelle erhalten. Da sie keine geeigneten Replikationsursprünge aufweist, wird sie nicht repliziert und bei der Zellteilung nicht gezielt auf die Tochterzelle weitergegeben (transiente Transfektion). Dieses alternative Verfahren ist vor allem dann von Vorteil, wenn auf der externen Nukleinsäure kodierte Gene in der Wirtszelle exprimiert werden, um anschließend in weiteren Versuchen analysiert zu werden. Weiterhin eignet sich dieses alternative Verfahren für die Untersuchung von Genen, deren Genprodukte (Proteine) für die Zelle toxisch sind, so daß die Zelle bei weiterer Zellkulturpassage nicht überleben würde.

In einer bevorzugten Ausführungsform wird als Ausgangszelle für die Transfektion insbesondere eine eukaryotische Zelle, vorzugsweise eine Säugerzelle und insbesondere eine menschliche Zelle, wie beispielsweise eine HeLa-Zelle verwendet.

Als Ausgangszellen für das erfindungsgemäße Verfahren können insbesondere adhärent wachsende Zellkultur-Zellen verwendet werden, die im ständigen Kontakt zu dem Kultivierungsgefäß, insbesondere auf dem Boden des Kultivierungsgefäßes in einem "Rasen" wachsen. Des weiteren können die Zellen adhärent auf Microcarriern wachsen. Bevorzugte Zellinien sind beispielsweise epidermale Zellinien wie HeLa- oder 293-Zellen.

In einer alternativen Ausführungsform können aber auch Zellen verwendet werden, die ohne ständigen Kontakt zu dem Kultivierungsgefäß im Zellkulturmedium wachsen und vermehrt werden, wie beispielsweise Blutzellen oder Zellen des lymphatischen Systems, die auch im natürlichen Organismus keinen Zell-Zell-Kontakt zum Wachstum benötigen und deren Wachstum richtungsunabhängig ist. Ferner sind Zellinien geeignet, die an das Wachstum in Suspensionskultur adaptiert wurden.

Ein weiterer Gegenstand der vorliegenden Erfindung ist ein in vitro Verfahren zur Herstellung einer transfizierten Zelle, wobei die wenigstens eine Nukleinsäure in wenigstens eine Zelle mittels CaPi nach dem erfindungsgemäßen Verfahren eingebracht wird, sowie die transfizierte Zelle, die mit Hilfe des erfindungsgemäßen Verfahrens erhalten wird.

Die transfizierte Zelle kann insbesondere zur Herstellung von rekombinanten A-deno-assoziierten Viren (rAAV) verwendet werden. Eine detaillierte Beschreibung der Herstellung der rAAV mit Hilfe des erfindungsgemäßen Verfahrens ist in den Beispielen beschrieben, wobei die Beispiele die Erfindung lediglich mehr erläutern sollen, ohne sie darauf zu beschränken.

### BEISPIELE

### 1. Verwendete Materialien

| | |
|---|---|
| HeLa-t Zellen | MediGene Master Cell Bank |
| 6-Loch (well)-Platten | NUNC; Cat.No. 152795 |
| T25-Zellkulturflaschen | NUNC; Cat.No. 136196 |
| T185-Zellkulturflaschen | NUNC; Cat.No. 144903 |
| Wannenstapel | NUNC; Cat.No. 164327 |
| | |
| Zellkulturmedium DMEM | LifeTechnologies; Cat.No. 41965-039 |
| Fötales Kälberserum (FKS) | PAA; Cat.No. A15-653 |
| FKS-Nordamerika | Gibco; Cat.No. 10084-169 |
| L-Glutamin | LifeTechnologies; Cat.No. 25030-024 |
| Antibiotikum/Antimykotikum (AB/AM) | PAA; Cat.No. P11-002 |
| Gentamycin | Gibco; Cat.No. 15750-037 |
| CaCl₂ | Sigma; C-7902 |
| H₂O | Sigma; W-3500 |
| 0,2 µm Luftfilter | NUNC; Gelman 4210 |
| 2x BBS-Puffer | 50 mM BES, 280 mM NaCl, 0,75 mM Na₂HPO₄, 0,75 mM NaH₂PO₄, pH 6,8-7,1 optimiert), |
| | |
| Luciferase-Reagenz | Promega E1501 |
| Triton-Lyse-Puffer | Promega E1501 |
| Luciferase-Meßgerät | Berthold Lumat LB 9501 |

### 2. Verwendete Plasmide

| | |
|---|---|
| Helferplasmid pSVori | Chiorini et al. (1995) Hum Gene |
| | Ther 6(12):1531 |
| Helferplasmid pUC"rep/fs/cap"(RBS)Δ37 | DE19905501 |
| Helferplasmid pUC"rep/cap"Δ37 | DE19905501 |
| Helferplasmid pUC"rep/cap"(RBS)Δ37 | DE19905501 |
| Transgenplasmip AAV-(B7.2free/GMCSF) | DE19905501 |
| pCI-luc-Plasmid | Beispiel 5 |
| Transgenplasmid pAAV-GFP | Beispiel 5 |
| Transgenplasmid pAAV-(B7.2) | Beispiel 5 |
| Adenovirus-5 | ATCC |

### 3. Transfektion unter CO₂-Begasung mit und ohne Temperatur- und CO₂-Shift

3x 10⁵ HeLa-t Zellen wurden in 3 ml DMEM-Vollmedium (500 ml DMEM + 50 ml FKS + 5 ml L-Glutamin + 5 ml AB/AM) jeweils in einem Loch (well) einer 6-well Platte ausgesät und für 16 - 24 h bei 37°C/5 % CO₂ inkubiert. Sämtliche Transfektionen wurden in Tripletts durchgeführt, wobei insgesamt vier verschiedene Transfektionen durchgeführt wurden: mit und ohne Shift der Temperatur bzw. der CO₂ Atmosphäre in Kombination mit und ohne Medienwechsel. Der Medienwechsel erfolgte direkt vor Zugabe der DNA-CaPi-Präzipitate. Das alte Medium wurde dabei gegen 3 ml frisches, unbegastes, vorgewärmtes Vollmedium ausgetauscht.

### DNA-CaPi-Präzipitatbildung:

Pro well wurden 6 µg pCI-luc DNA mit 150 µl 260 mM CaCl₂ gut gemixt und danach vorsichtig mit 150 µl 2x BBS gemischt. Die Lösung blieb 15 min bei Raumtemperatur ruhig stehen. Danach wurden pro well je 300 µl des DNA-CaPi-Gemisches zügig zu den 3 ml Medium in ein well pipettiert.

Die Proben ohne Temperatur/CO₂-Shift wurden direkt bei 37°C/5 % CO₂ inkubiert. Die Proben mit Shift wurden hingegen für 16-20 h bei 35°C/3 % CO₂ und erst danach bei 37°C/5% CO₂ inkubiert.

Nach insgesamt ca. 40-48 h Inkubation wurden die Zellen geerntet und die Luciferase-Expression gemessen. Dazu wurden die Zellen mit 500 µl PBS gewaschen, in 500 µl Triton-Lyse-Puffer bei Raumtemperatur lysiert und mit einem Zellschaber von der Oberfläche abgelöst. Das Lysat wurde in einem 1,5 ml Zentrifugenröhrchen aufgenommen und für 2 min bei maximaler Drehzahl abzentrifugiert. 2 µl des Überstandes wurden in 50 µl Luciferase-Reagenz gegeben und sofort gemessen.

Der Messwert wurde durch die Anzahl der transfizierten Zellen (= ausgesäte Zellen x2) geteilt und mit 1x 10⁶ multipliziert, so daß der Wert auf 1x 10⁶ Zellen bezogen ist.

**Tabelle 1 : Vergleich einer Transfektion mit (+) und ohne (-) Temperatur/CO₂₋Shift, ohne (-) und nach (+) Medienwechsel. Als Reportergen wurde das Luziferasegen (luc) verwendet. Die Meßwerte sind in relativen Luciferase-Einheiten (RLE) pro 1x 10⁶ Zellen ausgedrückt. Die Prozentwerte geben die Transfektionseffizienz verglichen mit der Standardmethode an (linke Spalte = +Shift -Medienwechsel = 100 %). Die Leerwerte sind bereits abgezogen. Es wurden jeweils 3 Versuchsansätze gemittelt.**

| **+ Temperatur/CO**_{**2**}**-Shift** | | **- Temperatur/CO**_{**2**}**-Shift** | |
|---|---|---|---|
| - Medienwechsel | + Medienwechsel | - Medienwechsel | + Medienwechsel |
| 9,12x10⁸ | 7,15x10⁸ | 6,93x10⁸ | 7,49x10⁸ |
| 100% | 78 % | 76% | 82% |

Dieses Experiment macht deutlich, daß die Standard-Methode mit Temperatur/CO₂-Shift und ohne Medienwechsel eine ähnliche Transfektionseffizienz zeigt wie die übrigen Versuchsansätze (siehe Tabelle 1).

### 4. Transfektion mit und ohne CO₂-Begasung, mit und ohne Temperatur-Shift

HeLa-t Zellen wurden in DMEM-Vollmedium (500 ml DMEM + 50 ml FKS + 5 ml L-Glutamin + 5 ml AB/AM) bei 37°C/5 % CO₂ kultiviert. 5x 10⁵ Zellen wurden pro T25-Flasche in 5 ml Vollmedium ausgesät. Die Zellen wurden für 16-24 h bei 37°C/5 % CO₂ inkubiert. Danach wurde das alte Medium gegen 3 ml frisches, unbegastes, angewärmtes Medium ausgetauscht.

### Ansatz des DNA-CaPi-Gemisches:

Pro T25 wurden 6 µg DNA mit 150 µl 260 mM CaCl₂ gemixt und anschließend mit 150 µl 2x BBS vorsichtig gemischt. Die Lösung blieb 15 min bei Raumtemperatur ruhig stehen. Danach wurden pro T25-Flasche je 300 µl des DNA-CaPi-Gemisches zügig zu den 3 ml Medium in der Flasche pipettiert.

Die Proben ohne Temperatur-Shift und ohne CO₂₋Begasung wurden in einem Inkubator bei 37°C ohne CO₂-Begasung inkubiert. Dazu wurden die Kappen zusätzlich mit drei Lagen Parafilm verschlossen. Die Proben mit Temperatur-Shift wurden für 16-20 h bei 35°C/3 % CO₂ und erst danach bei 37°C/5 % CO₂ inkubiert.

Nach insgesamt ca. 40-48 h Inkubation wurden die Zellen geerntet und die luc-Expression gemessen. Dazu wurden die Zellen mit 2000 µl PBS gewaschen, in 1000 µl Triton-Lyse-Puffer bei Raumtemperatur lysiert und mit einem Zellschaber von der Oberfläche abgelöst. Das Lysat wurde in einem 1,5 ml Zentrifugenröhrchen aufgenommen und für 2 min bei maximaler Drehzahl abzentrifugiert. 2 µl des Überstandes wurden in 50 µl Luciferase-Reagenz gegeben und sofort gemessen.

Der Messwert wurde durch die Anzahl der transfizierten Zellen (= ausgesäte Zellen x2) geteilt und mit 1x 10⁶ multipliziert, so daß man einen auf 1x 10⁶ Zellen bezogenen Wert erhält.

**Tabelle 2: Vergleich von Transfektionen mit (+) und ohne (-) Temperatur-Shift und mit (+) und ohne (-) CO₂-Begasung. Es wurden 2 unabhängige Experimente mit je 3 gemittelten Versuchsansätzen durchgeführt. Zellen ohne CO₂-Begasung wurden während der Transfektion CO₂-frei gehalten. Vor den Transfektionen erfolgte jeweils ein Medienwechsel. Die Meßwerte sind jeweils in RLE pro 1x 10⁶ Zellen ausgedrückt. Die Werte mit CO₂ und Shift wurden gleich 100 % gesetzt (= Standard).**

| **Versuch 1** | | **Versuch 2** | |
|---|---|---|---|
| **+ CO**_{**2**} **+ Temp.-Shift** | **- CO**_{**2**} **- Temp.-Shift** | **+ CO**_{**2**} **+ Temp-Shift** | **-CO2 - Temp.-Shift** |
| 5,64x10⁸ | 5,06x10⁸ | 2,54x10⁸ | 2,76x10⁸ |
| 100% | 90% | 100% | 109% |

Diese Ergebnisse zeigen, daß die Transfektion ohne CO₂-Begasung und ohne Temperatur-Shift ebenso effizient ist wie die herkömmliche Methode mit CO₂₋Begasung und mit Temperatur-Shift (siehe Tabelle 2).

### 5. Klonierung der Plasmide:

### 1) pCI-luc wurde wie folgt kloniert:

Der Vektor pBL (Hoppe-Seyler et al. (1991) J. Virol. 65:5613-; Butz und Hoppe-Seyler (1993) J. Virol. 67:6476) wurde mit den Restriktionsenzymen SmaI und Pvu II geschnitten, um das Luciferasegen zu isolieren (Insert). Der Vektor pCI (Promega GmbH, Mannheim, Deutschland) wurde mit SmaI linearisiert und dephosphoryliert (Vektor). Beide Fragmente - Insert und Vektor - wurden zu pCI-Luc ligiert.

### 2) pAAV-GFP wurde wie folgt hergestellt:

Ausgangsplasmid für die Subklonierung des "green fluorescence protein" (GFP) war der Vektor pEGFP-N3 (Clontech, Heidelberg, Deutschland). Mit den Oligonukleotiden (Primern) GFP/SpeI und GFP/MluI wurde über Polymerasekettenreaktion (PCR) ein etwa 3000 bp großes Fragment amplifiziert, welches mit Spe I und Mlu I nachgeschnitten wurde. Dieses Fragment wurde in den AAV Basisvektor einkloniert, wodurch pAAV-GFP generiert wurde. Die Herstellung des Basisvektors ist in der DE19905501 beschrieben. (In Kürze: pAV2 (Laughlin et al. (1983) Gene 23:65) wurde mit Bgl II geschnitten und das AAV Fragment in pUC 19 über die BamHI-Schnittstelle eingesetzt. In dem so entstandenen pUCAV2 wurden die AAV Nukleotide 192-4497 deletiert und durch die pCI Nukleotide 4002-4008 sowie 1-1351, welche die CMV-polyA Expressionskassette beinhalten, ersetzt. Dieses Konstrukt wird mit AAV Basisvektor bezeichnet).

### Primersequenzen:

GFP/SpeI: 5'- GGG ATC CAT CAC TAG TAT GGT GAG CAA GG - 3'
GFP/MluI: 5'- CAA ACG ACC CAA CAC CAC GCG TTT TAT TCT GTC - 3'

### 3) pAAV-(B7.2) wurde wie folgt über zwei Klonierungsschritte hergestellt:

Zunächst wurden die Vektoren pCI, und pLL279 (Lanier et. al. (1995) J Immunol 154(1):97) mit den Restriktionsenzymen Xho I und Not I geschnitten und das B7.2 Insert aus pLL279 mit dem pCI Vektor zu pCI-B7.2 ligiert. Mit den Primern B7.2/NheI und B7.2/MluI wurde aus pCI-B7.2 über PCR ein etwa 3000 bp großes Fragment amplifiziert, welches mit Nhe I und Mlu I nachgeschnitten wurde. Dieses Fragment wurde in den AAV Basisvektor (siehe Beispiel 5.2) einkloniert, wodurch pAAV-(B7.2) generiert wurde.

### Primersequenzen:

B7.2/NheI: 5'- GCA TTT GTG CTA GCA CTA TGG GAC TGA G - 3'
B7.2/MluI: 5'- CGG TTC ACG CGT ATC AAG GCG AGT TAC ATG - 3'

### 6. Verpackung von rAAV-GFP (Zellkultur in T25-Zellkulturflaschen ohne CO₂ Begasung

HeLa-t Zellen wurden in DMEM-Vollmedium (500 ml DMEM + 50 ml FKS + 5 ml L-Glutamin + 5 ml AB/AM) bei 37°C/5 % CO₂ kultiviert. 5x 10⁵ Zellen wurden pro T25-Zellkulturflasche (T25) in 5 ml Vollmedium ausgesät. Die Zellen wurden für 16-24 h bei 37°C/5 % CO₂ inkubiert. Danach wurde das alte Medium gegen 3 ml frisches, unbegastes, angewärmtes Medium ausgetauscht.

### Ansatz des DNA-CaPi-Gemisches (für Co-Transfektion):

Pro T25 wurden 2 µg pAAV-GFP- mit 4 µg pSVori-DNA und 150 µl 260 mM CaCl₂ gemixt und anschließend mit 150 µl 2x BBS vorsichtig gemischt. Die Lösung blieb 15 min bei Raumtemperatur ruhig stehen. Danach wurden pro T25 je 300 µl des DNA-CaPi-Gemisches zügig zu den 3 ml Medium in der Flasche pipettiert.

Die Proben ohne Temperatur-Shift und ohne CO₂-Begasung wurden in einem Inkubator bei 37°C ohne CO₂-Begasung inkubiert. Dazu wurden die Kappen zusätzlich mit zwei Lagen Parafilm verschlossen. Die anderen Ansätze wurden für 16-20 h bei 35°C/3 % CO₂ und erst danach bei 37°C/5 % CO₂ inkubiert.

Nach insgesamt ca. 40-48 h Inkubation wurden die Zellen mit Adenovirus infiziert. Dazu wurde das Medium aus den Flaschen entfernt und durch 1,5 ml frisches Vollmedium ersetzt. Die Adenovirus-Lösung wurde auf 2x 10⁹ Partikel/ml verdünnt. Je Flasche wurden 50 µl des verdünnten Virus zu den 1,5 ml Medium geben. Die Flaschen wurden verschlossen und für 2 h entsprechend inkubiert. Danach wurden 3 ml frisches Vollmedium zugegeben und für weitere 20 h inkubiert. Es folgte ein erneuter Austausch des alten Mediums gegen 5 ml neues Medium.

Anschließend wurde (entsprechend mit bzw. ohne CO₂, also ohne und mit zusätzlichem Parafilmverschluß) für weitere 60-70 h inkubiert. Danach werden die Zellen und der Überstand geerntet. Dazu wurden letzte noch anhaftende Zellen mit Überstand vom Flaschenboden abgespült und in einem 15 ml Spitzbodenzentrifugenröhrchen aufgenommen. Schließlich wurden die Zellen durch dreimaliges Einfrieren und Auftauen der Zellen (in flüssigem N₂ bzw. bei 37°C im Wasserbad) lysiert.

### Titerbestimmung:

Zur Titerbestimmung wurden 1,5x 10⁵ HeLa-t Zellen in je einem well einer 12well-Platte ausgesät und in 1 ml Vollmedium 16-20 h inkubiert (37°C/5 % CO₂). Danach wurden die Zellen durch γ- oder UV-Bestrahlung inaktiviert. Das Viruslysat wurde 10 min bei 60 °C hitzeinaktiviert, anschließend abzentrifugiert und der Überstand für die Virustitration verwendet. Dazu wurden die Zellen mit verschiedenen Mengen des Lysats infiziert und für 40-48 h bei 37°C/5 % CO₂ inkubiert. Danach wurden die Zellen geerntet, wobei der Überstand abgenommen, die Zellen mit PBS gewaschen und durch EDTA/Trypsin-Behandlung von dem Zellkulturgefäß abgelöst wurden. Mittels "fluorescence activated cell sorting" (FACS) wurde schließlich der Prozentsatz der GFP-positiven Zellen und so der Virustiter bestimmt.

**Tabelle 3: Die Zellen wurden wie unter 4. beschrieben mit dem Transgenplasmid pAAV-GFP und dem Helferplasmid pSVori co-transfiziert (n=39). Die weitere Verpackung erfolgte standardmäßig, jedoch bei - CO₂ - Temp.-Shift weiterhin ohne CO₂-Begasung. Die Werte des Standards wurden gleich 100 % gesetzt. Als Maß wurde die Menge des pro Zelle produzierten Virus bestimmt.**

| | **+ CO**_{**2**} **+ Temp.-Shift** | **- CO**_{**2**} **- Temp.-Shift** |
|---|---|---|
| Transduzierende Partikel pro Zelle | 12 | 14 |
| Leistungsindex | 100 % | 117 % |

Demnach ist die Verpackung von rAAV-GFP ohne CO₂-Begasung und ohne Temperatur-Shift vergleichbar effizient wie mit CO₂-Begasung und mit Temperatur-Shift (siehe Tabelle 3).

### 7. Vergleich von Verpackungen in Wannenstapeln (WS) mit und ohne CO₂

HeLa-t Zellen wurden in T185-Zellkulturflaschen bei 37°C/5 % CO₂ kultiviert. Die Zellen wurden mit EDTA/Trypsin geerntet und in DMEM-Vollmedium (500 ml DMEM + 50 ml FKS +5 ml 200 mM L-Glutamin) resuspendiert (Zugabe von 50 µg/ml Gentamycin möglich). 0,9x10⁸ bis 1,4x10⁸ HeLa-t Zellen wurden in 900 ml Vollmedium aufgenommen. Mit dieser Zellsuspension wurde ein Wannenstapel befüllt. Zur Transfektion ohne CO₂-Begasung und ohne Temperatur-Shift wurden die beiden Wannenstapelanschlüsse mit Parafilm oder einem luftdicht verschließbaren Luftfilteraufsatz verschlossen. Die Inkubation erfolgte für 22 - 30 h bei 37°C ohne CO₂. Die Ansätze mit CO₂-Begasung und mit Temperatur-Shift wurden für den gleichen Zeitraum bei 37°C/5 % CO₂ inkubiert.

Nach der Inkubation wurde der Überstand abgenommen. Es folgte die erste Transfektion mit dem jeweiligen Helferplasmid (siehe Tabelle 4). Dazu wurden 1800 µg DNA in 45 ml 270 mM CaCl₂ gelöst und gut gemischt. Danach wurden 45 ml 2x BBS zugegeben und vorsichtig gemischt. Das DNA-CaPi-Gemisch wurde 20 ⁺/- 5 min in Ruhe bei Raumtemperatur stehengelassen. Anschließend wurden die 90 ml DNA-CaPi-Lösung zügig zu 900 ml vorgewärmten DMEM-Vollmedium (mit oder ohne Gentamycin) gegeben. Das Medium wurde mit den Präzipitaten in den Wannenstapel überführt und die Zellen erneut für 20 - 30 h bei 37°C ohne Begasung inkubiert.

Danach erfolgte die Transfektion des jeweiligen Transgenplasmids (siehe Tabelle 4). Diese erfolgte genau wie für das Helferplasmid beschrieben.

**Tabelle 4: Kombination der verwendeten Helferplasmide und Transgenplasmide der Experimente aus Tabelle 5 (siehe unten).**

| **Helferplasmid** | **Transgenplasmid** | **Anzahl an Experimenten** |
|---|---|---|
| PUC"rep/fs/cap"(RBS)Δ37 | pAAV-GFP | 1 |
| PUC"rep/fs/cap"(RBS)Δ37 | pAAV-(B7.2) | 1 |
| PUC"rep/cap"(RBS)Δ37 | pAAV-(B7.2free/GMCSF) | 1 |
| PUC"rep/cap"Δ37 | pAAV-(B7.2free/GMCSF) | 2 |

Nach 20 - 30 h Inkubation wurde Adenovirus mit einer Infektionseinheit (multiply of infectivity (MOI)) von 2 - 3 zu den Zellen gegeben. Nach weiteren 20 - 30 h Inkubation bei 37°C ohne Begasung wurde der Überstand gegen 900 ml frisches Medium ohne FKS ausgetauscht. Nach diesem letzten Medienwechsel wurde der Wannenstapel für weitere 60 -68 h bei 37°C inkubiert. Danach erfolgte die Ernte des Virus durch Frier/Tau-Lyse der Zellen.

Im Vergleich dazu erfolgte bei der Standardmethode vor der Transfektion mit dem Helferplasmid kein Medienwechsel. Außerdem wurden die Zellen dabei durch einen 0,2 µm Luftfilter begast. Nach Zugabe der DNA-CaPi-Gemische wurden die Zellen zunächst für eine Nacht bei 35°C/3 % CO₂ inkubiert, bevor sie auf 37°C/5 % CO₂ geshiftet wurden. Bis auf diese Unterschiede waren beide Methoden identisch.

**Tabelle 5: Vergleich von Wannenstapeln (WS), die mit (+) und ohne (-) CO₂₋Begasung/Temperatur-Shift zur Verpackung verschiedener Vektoren verwendet wurden. In fünf Experimenten wurden WS mit und ohne CO₂/Temperatur-Shift direkt miteinander verglichen (= ansonsten identische und zeitgleiche Herstellung). Gemessen wurde die Anzahl der transduzierenden Partikel pro Wannenstapel.**

| | **+ CO**_{**2**} **+ Temp.-Shift** | **-CO**_{**2**} **-Temp.-Shift** |
|---|---|---|
| **Anzahl Experimente** | 5 | 5 |
| **durchschnittl. transduzierte Partikel/WS** | 9,75x10⁹ | 10,01x10⁹ |

Auch in Wannenstapeln, die sich für die großtechnische Kultivierung von adhärent wachsenden Zellen eignen, sind die Ausbeuten bei der Herstellung von rekombinanten AAV ohne CO₂-Begasung und ohne Temperatur-Shift vergleichbar mit der Standard-Methode mit CO₂-Begasung und mit Temperatur-Shift (siehe Tabelle 5).

## Patentansprüche

1. Verfahren in vitro zum Einbringen von wenigstens einer Nukleinsäure in wenigstens eine Zelle mittels Calciumphosphat (CaPi), das die Schritte umfaßt:
(a) Vermischen der genannten Nukleinsäure oder Nukleinsäuren mit mindestens einer Reaktionslösung, die Ca²⁺ und PO₄³⁻ -Ionen aufweist;
(b) Inkubieren der Reaktionlösung(en), wodurch ein Präzipitat gebildet wird, das wenigstens Nukleinsäure, Calcium und Phosphat aufweist;
(c) Zugeben des Präzipitats zu der genannten Zelle;
(d) Inkubieren der Zelle aus Schritt (c) unter Luft im geschlossenen System ohne zusätzliches Kohlendioxid (CO₂), wodurch die Nukleinsäure oder Nukleinsäuren in die Zelle aufgenommen wird.

2. Verfahren nach Anspruch 1, **dadurch gekennzeichnet, daß** als Nukleinsäure RNA oder DNA, vorzugsweise genomische DNA, cDNA, lineare DNA oder Plasmid-DNA, insbesondere zirkuläre Plasmid-DNA verwendet wird.

3. Verfahren nach Anspruch 1 oder 2, **dadurch gekennzeichnet, daß** die Nukleinsäure in einer Menge von 0,2 µg - 20 µg, vorzugsweise von 1 µg - 20 µg, insbesondere von 2 µg - 10 µg, vor allem von 6 µg pro 10⁶ Zellen verwendet wird.

4. Verfahren nach einem der Ansprüche 1 bis 3, **dadurch gekennzeichnet, daß** gleichzeitig oder nacheinander zwei oder mehrere verschiedene Nukleinsäuren, vorzugsweise zwei oder drei verschiedene Nukleinsäuren, insbesondere zwei oder drei verschiedene Plasmid-DNAs verwendet werden.

5. verfahren nach einem der Ansprüche 1 bis 4, **dadurch gekennzeichnet, daß** der pH-Wert der Reaktionslösung in Schritt (b) bei Raumtemperatur 6,8-7,1, vorzugsweise 6,86 - 7,05, insbesondere 7,05 beträgt.

6. Verfahren nach einem der Ansprüche 1 bis 5, **dadurch gekennzeichnet, daß** das Inkubieren der Reaktionslösung in Schritt (b) bei Raumtemperatur für 5 - 30 min, vorzugsweise für 10 - 20 min, insbesondere für 15 min erfolgt.

7. Verfahren nach einem der Ansprüche 1 bis 6, **dadurch gekennzeichnet, daß** das Inkubieren der Zellen in Schritt (d) bei 37°C für 20 - 72 h, vorzugsweise 30-72 h, insbesondere für 24 - 48 h, vor allem 40-48 h erfolgt.

8. Verfahren nach einem der Ansprüche 1 bis 7, **dadurch gekennzeichnet, daß** die aufgenommene Nukleinsäure einen Replikationsursprung aufweist, episomal in der transfizierten Zelle erhalten bleibt und bei der Zellteilung an die Tochterzelle kontrolliert weitergegeben wird.

9. Verfahren nach einem der Ansprüche 1 bis 7, **dadurch gekennzeichnet, daß** die aufgenommene Nukleinsäure keinen Replikationsursprung aufweist und gerichtet oder ungerichtet in das Genom der Zelle integriert und **dadurch** bei der Zellteilung an die Tochterzelle weitergegeben wird.

10. Verfahren nach einem der Ansprüche 1 bis 7, **dadurch gekennzeichnet, daß** die aufgenommene Nukleinsäure keinen Replikationsursprung aufweist und somit bei der Zellteilung nicht gezielt an die Tochterzelle weitergegeben wird.

11. Verfahren nach einem der Ansprüche 1 bis 10, **dadurch gekennzeichnet, daß** als Zelle eine eukaryotische Zelle, vorzugsweise eine Säugetierzelle, insbesondere eine menschliche Zelle verwendet wird.

12. Verfahren nach einem der Ansprüche 1 bis 11, **dadurch gekennzeichnet, daß** die Zelle in ständigem Kontakt zu dem Kultivierungsgefäß im Zellkulturmedium wächst und sich vermehrt.

13. Verfahren nach einem der Ansprüche 1 bis 11, **dadurch gekennzeichnet, daß** die Zelle ohne ständigen Kontakt zu dem Kultivierungsgefäß im Zellkulturmedium wächst und sich vermehrt.

14. Verfahren in vitro zur Herstellung einer transfizierten Zelle, **dadurch gekennzeichnet, daß** wenigstens eine Nukleinsäure in wenigstens eine Zelle mittels CaPi gemäß dem Verfahren nach einem der Anspruche 1 bis 13 eingebracht wird.

## Claims

1. *In vitro* method of introducing at least one nucleic acid into at least one cell by means of calcium phosphate (CaPi) comprising the steps:
(a) mixing the mentioned nucleic acid or nucleic acids with at least one reaction solution that contains Ca²⁺ and PO₄³⁻ ions;
(b) incubating the reaction solution(s), so that a precipitate is formed which comprises at least nucleic acid, calcium and phosphate;
(c) adding the precipitate to the mentioned cell;
(d) incubating the cell from step (c) under air in a closed system without additional carbon dioxide (CO₂) so that the nucleic acid or nucleic acids is absorbed into the cell.

2. Method according to claim 1, **characterised in that** the nucleic acid used is RNA or DNA, preferably genomic DNA, cDNA, linear DNA or plasmid DNA, especially circular plasmid DNA.

3. Method according to claim 1 or 2, **characterised in that** the nucleic acid is used in an amount of from 0.2 µg to 20 µg, preferably from 1 µg to 20 µg, especially from 2 µg to 10 µg, more especially 6 µg, per 10⁶ cells.

4. Method according to any one of claims 1 to 3, **characterised in that** two or more different nucleic acids, preferably two or three different nucleic acids, especially two or three different plasmid DNAs, are used simultaneously or in succession.

5. Method according to any one of claims 1 to 4, **characterised in that** the pH value of the reaction solution in step (b) at room temperature is from 6.8 to 7.1, preferably from 6.86 to 7.05, especially 7.05.

6. Method according to any one of claims 1 to 5, **characterised in that** the incubation of the reaction solution in step (b) takes place at room temperature for from 5 to 30 min, preferably for from 10 to 20 min, especially for 15 min.

7. Method according to any one of claims 1 to 6, **characterised in that** the incubation of the cells in step (d) takes place at 37°C for from 20 to 72 hours, preferably from 30 to 72 hours, especially for from 24 to 48 hours, more especially from 40 to 48 hours.

8. Method according to any one of claims 1 to 7, **characterised in that** the absorbed nucleic acid has an origin of replication, is retained episomally in the transfected cell and is passed on to the daughter cell in a controlled manner during cell division.

9. Method according to any one of claims 1 to 7, **characterised in that** the absorbed nucleic acid has no origin of replication and is integrated into the genome of the cell in a targeted or non-targetted manner and is thereby passed on to the daughter cell during cell division.

10. Method according to any one of claims 1 to 7, **characterised in that** the absorbed nucleic acid has no origin of replication and accordingly is not passed on specifically to the daughter cell during cell division.

11. Method according to any one of claims 1 to 10, **characterised in that** the cell used is a eukaryotic cell, preferably a mammal cell, especially a human cell.

12. Method according to any one of claims 1 to 11, **characterised in that** the cell grows and multiplies in the cell culture medium while being in constant contact with the culturing vessel.

13. Method according to any one of claims 1 to 11, **characterised in that** the cell grows and multiplies in the cell culture medium without being in constant contact with the culturing vessel.

14. *In vitro* method for the preparation of a transfected cell, **characterised in that** at least one nucleic acid is introduced into at least one cell by means of CaPi in accordance with the method according to any one of claim 1 to 13.

## Revendications

1. Procédé *in vitro* pour introduire au moins un acide nucléique dans au moins une cellule à l'aide de phosphate de calcium (CaPi), qui comprend les étapes consistant à :
(a) mélanger l'acide nucléique ou les acides nucléiques mentionnés avec au moins une solution réactionnelle qui présente des ions Ca²⁺ et PO₄³⁻ ;
(b) incuber la ou les solutions réactionnelles, si bien qu'il se forme un précipité qui présente au moins un acide nucléique, du calcium et du phosphate ;
(c) ajouter le précipité à la cellule mentionnée ;
(d) incuber la cellule de l'étape (c) sous air dans un système fermé sans dioxyde de carbone (CO₂) supplémentaire, si bien que l'acide nucléique ou les acides nucléiques sont absorbés dans la cellule.

2. Procédé selon la revendication 1, **caractérisé en ce que** l'on utilise, en tant qu'acide nucléique, de l'ARN ou de l'ADN, de préférence de l'ADN génomique, de l'ADNc, de l'ADN linéaire ou de l'ADN plasmidique, en particulier de l'ADN plasmidique circulaire.

3. Procédé selon la revendication 1 ou 2, **caractérisé en ce que** l'on utilise l'acide nucléique en une quantité de 0,2 µg à 20 µg, de préférence de 1 µg à 20 µg, en particulier de 2 µg à 10 µg, principalement de 6 µg pour 10⁶ cellules.

4. Procédé selon l'une quelconque des revendications 1 à 3, **caractérisé en ce que** l'on utilise simultanément ou successivement deux acides nucléiques différents ou plus, de préférence deux ou trois acides nucléiques différents, en particulier deux ou trois ADN plasmidiques différents.

5. Procédé selon l'une quelconque des revendications 1 à 4, **caractérisé en ce que** le pH de la solution réactionnelle à l'étape (b) est, à température ambiante, de 6,8 à 7,1, de préférence de 6,86 à 7,05, en particulier de 7,05.

6. Procédé selon l'une quelconque des revendications 1 à 5, **caractérisé en ce que** l'incubation de la solution réactionnelle à l'étape (b) est réalisée à température ambiante pendant 5 à 30 minutes, de préférence 10 à 20 minutes, en particulier 15 minutes.

7. Procédé selon l'une quelconque des revendications 1 à 6, **caractérisé en ce que** l'incubation des cellules à l'étape (d) est réalisée à 37°C pendant 20 à 72 heures, de préférence 30 à 72 heures, en particulier 24 à 48 heures, principalement 40 à 48 heures.

8. Procédé selon l'une quelconque des revendications 1 à 7, **caractérisé en ce que** l'acide nucléique absorbé présente une origine de réplication, est maintenu sous forme épisomique dans la cellule transfectée et est transmis à la cellule fille de manière maîtrisée lors de la division cellulaire.

9. Procédé selon l'une quelconque des revendications 1 à 7, **caractérisé en ce que** l'acide nucléique absorbé ne présente pas d'origine de réplication et est intégré de manière dirigée ou non dirigée dans le génome de la cellule et est ainsi transmis à la cellule fille lors de la division cellulaire.

10. Procédé selon l'une quelconque des revendications 1 à 7, **caractérisé en ce que** l'acide nucléique absorbé ne présente pas d'origine de réplication et est donc transmis à la cellule fille de manière non ciblée lors de la division cellulaire.

11. Procédé selon l'une quelconque des revendications 1 à 10, **caractérisé en ce que** l'on utilise, en tant que cellule, une cellule eucaryote, de préférence une cellule de mammifère, en particulier une cellule humaine.

12. Procédé selon l'une quelconque des revendications 1 à 11, **caractérisé en ce que** la cellule se développe et se multiplie en contact permanent avec le récipient de culture dans le milieu de culture de cellules.

13. Procédé selon l'une quelconque des revendications 1 à 11, **caractérisé en ce que** la cellule se développe et se multiplie sans contact permanent avec le récipient de culture dans le milieu de culture de cellules.

14. Procédé *in vitro* de production d'une cellule transfectée, **caractérisé en ce qu'**au moins un acide nucléique est introduit dans au moins une cellule à l'aide de CaPi au moyen du procédé selon l'une quelconque des revendications 1 à 13.


### SEQUENCE LISTING

<110> MediGene Aktiengesellschaft
<120> Verfahren in vitro zum Einbringen von Nukleinsäure in eine Zelle (Transfektion) mittels Calciumphosphat
<130> M32752PC
<140> PCT/EP 01/05531
   <141> 2001-05-15
<150> DE 10024334.7
   <151> 2000-05-17
<160> 4
<170> PatentIn version 3.1
<210> 1
   <211> 29
   <212> DNA
   <213> Künstliche Sequenz
<220>
   <223> Primer
   <400> 1
   gggatccatc actagtatgg tgagcaagg 29
<210> 2
   <211> 33
   <212> DNA
   <213> Künstliche Sequenz
<220>
   <223> Primer
   <400> 2
   caaacgaccc aacaccacgc gttttattct gtc 33
<210> 3
   <211> 28
   <212> DNA
   <213> Künstliche Sequenz
<220>
   <223> Primer
   <400> 3
   gcatttgtgc tagcactatg ggactgag 28
<210> 4
   <211> 30
   <212> DNA
   <213> Künstliche Sequenz
<220>
   <223> Primer
   <400> 4
   cggttcacgc gtatcaaggc gagttacatg 30